# EUROPEAN PATENT SPECIFICATION

(11) **EP 1 610 682 B1**
(45) Date of publication and mention of the grant of the patent: **11.08.2010**
(21) Application number: 04718680.4
(22) Date of filing: 09.03.2004
(51) Int. Cl.: A61B 5/15

(54) **IMPROVEMENTS RELATING TO SKIN PRICKERS**
VERBESSERUNGEN AN HAUT-PRICK-GERÄTEN
DISPOSITIF AMELIORE SERVANT A PERFORER LA PEAU

(30) Priority: 10.03.2003 GB 0305363
(43) Date of publication of application: 04.01.2006
(73) Proprietor: OWEN MUMFORD LIMITED, Woodstock, Oxfordshire OX20 1TU (GB)
(72) Inventor: MARSHALL, Jeremy, Oxford OX2 6DD (GB); MUMFORD, Adam, John, Oxford OX8 6EY (GB)
(74) Representative: Newell, William Joseph
(86) International application number: PCT/GB2004/000966
(87) International publication number: WO 2004/080305

(56) References cited:
- WO-A-02/43591

## Description

This invention relates to skin prickers. It is a development of that described in WO 02/043,591, which is concerned with ensuring that the lancet, once fired, cannot be pushed back via the needle tip aperture, re-cocked and refired. The present invention improves upon that design.

According to the present invention there is provided a disposable skin pricker comprising an elongate housing with a spring-loaded, longitudinally movable lancet carried therein, the lancet tip normally being within the housing, a trigger mechanism to retain the lancet in a fully retracted position in which it energises the spring, the trigger mechanism being actuable to release or fire the lancet to cause the tip to have a momentary position projecting from the forward end of the housing, and means for preventing repeated use including a spring finger extending rearwardly from the lancet alongside but spaced from the body thereof, and an abutment on the inside of the housing past which the tip of the finger will snap during forward motion of the lancet, any attempt to push the lancet back with a greater than a predetermined force after firing resulting in the abutment deflecting the finger tip out through an aperture in the side wall of the housing.

Preferably, the lancet is symmetrical, with two fingers on opposite sides thereof. The housing will then have two opposed apertures and abutments, and these abutments may be shaped as barbs pointing inwards and forwards.

In one form the or each finger inclines outwardly from the lancet body as well as extending rearwardly.

Alternatively the or each finger may be generally parallel to and spaced from the adjacent part of the body of the lancet but with its tip flaring outwardly.

The needle tip may initially be protected by an elongate cap by which the lancet can be pushed back to the fully retracted position from an initial pre-fired position wherein the or each finger tip is immediately to the rear of the associated abutment.

For a better understanding of the invention, one embodiment thereof will now be described, by way of example, with reference to the accompanying drawings, in which:
Figure 1 is a side view of a skin pricker of the invention;
Figure 2 is an axial section of the skin pricker of Figure 1 in an initial state;
Figure 3 is a similar section showing the lancet of the skin pricker in a cocked condition;
Figure 4 is an axial section through the skin pricker after its lancet has been fired;
Figure 5 is a similar section showing the skin pricker of Figure 4 after an attempt has been made to re-cock the lancet.

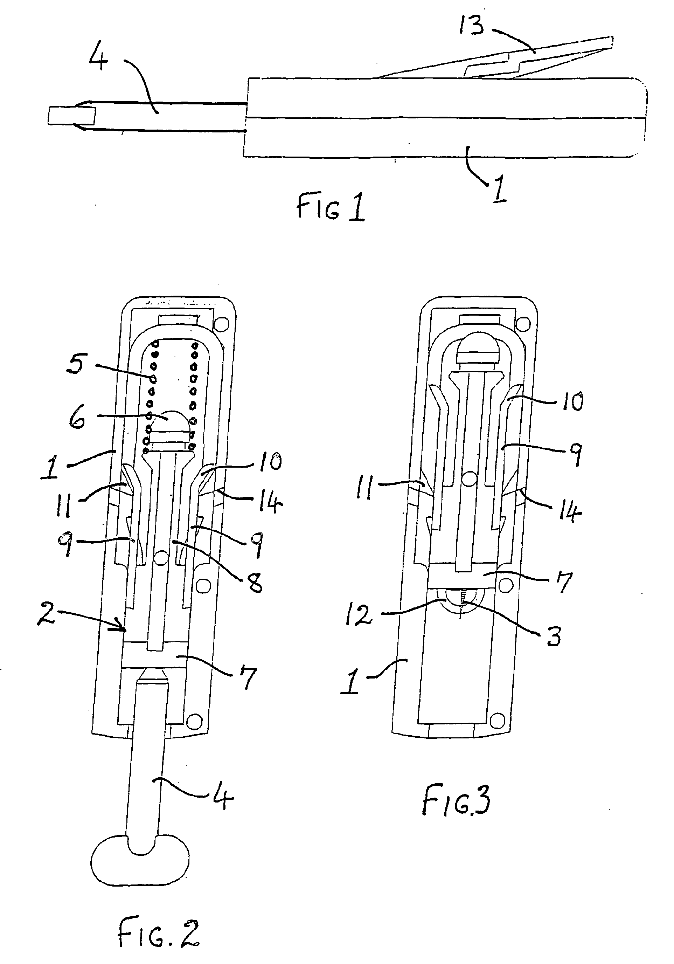

The device of Figures 1 and 2 has a barrel 1 of two halves joined at a longitudinal split to hold a lancet 2. The lancet has a plastics body encasing a needle 3 (Figure 3) whose tip is initially embedded in a twist-off elongate cap 4 moulded integrally with the body. A spring 5 which drives the lancet forwards is shown only in Figure 2. The spring acts on the head 6 of the lancet 2.

The lancet body has a large end portion 7 non-rotatably guided in the forward part of the barrel 1. A stem 8 extends rearwardly from the portion 7 terminating in the head 6. At opposite sides of the stem fingers 9 lead outwardly and rearwardly from the roots of the shoulders at the transition between the portion 7 and the stem 8. The fingers 9 are integrally moulded with the plastics body, and are resiliently flexible and can act in a springlike fashion. The fingers 9 have outwardly splayed tips 10. At about its mid-length the interior of the barrel 1 are formed inwardly and forwardly angled barbs 11.

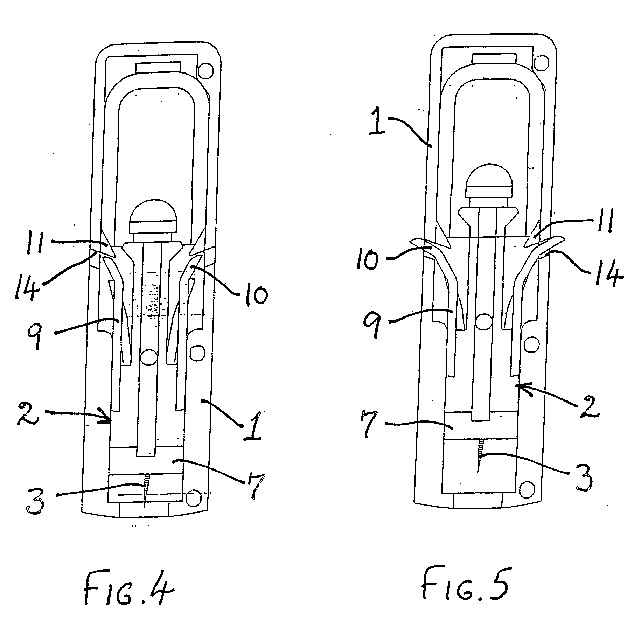

Initially, with the spring relaxed, the tips 10 of the fingers 9 are behind the barbs 11. The cap 4 is pressed to retract the lancet, and the device is then cocked, as shown in Figure 3, where the end portion 7 is locked behind a lug 12 of a trigger lever 13 (Figure 1). The cap is twisted off and the device is applied and fired by pressing the trigger 13. The thrust of the spring urges the lancet forwards and the fingers 9 flex inwards as the tips 10 snap past the barbs 11, just before the needle tip momentarily emerges from the forward end of the barrel. As the over-extended spring 5 retracts, the tips 10 of the arms 9 engage behind the barbs 11 (the condition shown in Figure 4). There is effectively a ratchet mechanism.

If the cap 4 is now used to try to push the lancet back into the cocked condition, while the main body of the lancet will move, at least initially, the fingers 9 are trapped. The fingers will flex as the tips 10 press against the barbs 11 until the arms bend and the tips 10 are pushed out through openings 14 in the side walls of the barrel 1 (the condition of Figure 4). Thus the lancet is arrested and immobilised before the cocked position is reached, and re-firing is prevented.

In the embodiment described, the lancet is symmetrical, with spring arms 9 on opposite sides. While this is preferred, it would be possible to construct and guide the lancet in a manner such that only one arm would suffice.

## Claims

1. A disposable skin pricker comprising an elongate housing (1) with a spring-loaded, longitudinally movable lancet (2) carried therein, the lancet tip (3) normally being within the housing (1), a trigger mechanism (13) to retain the lancet in a fully retracted position in which it energises the spring (5), the trigger mechanism being actuable to fire the lancet to cause the tip to have a momentary position projecting from the forward end of the housing, and means for preventing repeated use including a spring finger (9) extending rearwardly from the lancet alongside but spaced from the body thereof, and an abutment (11) on the inside of the housing past which the tip (10) of the finger (9) snaps when the lancet is fired, **characterised in that** an aperture (14) is provided in the side wall of the housing (1), whereby any attempt to push the lancet back with a greater than a predetermined force after firing results in the abutment (11) deflecting the finger tip (10) out through said aperture (14).

2. A disposable pricker according to claim, 1, wherein the lancet (2) is symmetrical, with two fingers (9) on opposite sides thereof.

3. A disposable pricker according to claim 2, wherein the housing has two opposed apertures (14) and abutments (11).

4. A disposable pricker according to claim 2, wherein the abutments (11) are shaped as barbs pointing inwards and forwards.

5. A disposable pricker according to any one of claims 1 to 4, wherein the or each finger (9) inclines outwardly from the lancet body as well as extending rearwardly.

6. A disposable pricker according to any one of claims 1 to 4, wherein the or each finger (9) is generally parallel to and spaced from the adjacent part of the body of the lancet but with its tip (10) flaring outwardly.

7. A disposable pricker according to any one of claims 1 to 4, wherein the needle tip (3) is initially protected by an elongate cap (4) by which the lancet (2) can be pushed back to the fully retracted position from an initial pre-fired position in which the or each finger tip (10) is immediately to the rear of the associated abutment.

## Patentansprüche

1. Wegwerfbarer Hautstecher mit einem länglichen Gehäuse, in dem eine federbelastete, in Längsrichtung angeordnete Lanzette (2) getragen wird, wobei die Lanzettenspitze (3) sich normalerweise in dem Gehäuse (1) befindet, ferner mit einem Auslösemechanismus (13) zum Zurückhalten der Lanzette in einer vollständig zurückgezogenen Lage, in der sie die Feder (5) spannt, wobei der Auslösemechanismus betätigbar ist, um die Lanzette abzuschießen, so daß die Spitze veranlaßt wird, eine kurzzeitige Position einzunehmen, in der sie aus dem vorderen Ende des Gehäuses herausragt, und wobei Mittel vorhanden sind, die eine wiederholte Benutzung verhindern, einschließlich eines Druckfingers (9), der sich längs der Lanzette von ihr nach hinten erstreckt, jedoch von ihrem Körper mit Abstand getrennt ist, sowie eines Anschlags (11) auf der Innenseite des Gehäuses (1), an dem die Spitze (10) des Druckfingers (9) vorbeischnappt, wenn die Lanzette abgefeuert wird, **dadurch gekennzeichnet, daß** eine Öffnung (14) in der Seitenwand des Gehäuses (1) vorgesehen ist, durch die jeglicher Versuch, die Lanzette mit einer größeren als einer vorbestimmten Kraft nach dem Abfeuern zurückzudrücken, dazu führt, daß der Anschlag (11) die Fingerspitze durch die Öffnung (14) nach außen biegt.

2. Wegwerfbarer Stecher nach Anspruch 1, **dadurch gekennzeichnet, daß** die Lanzette (2) symmetrisch ist und auf ihren entgegengesetzten Seiten zwei Finger (9) aufweist.

3. Wegwerfbarer Stecher nach Anspruch 2, **dadurch gekennzeichnet, daß** das Gehäuse zwei entgegengesetzte Öffnungen (14) und Anschläge (11) aufweist.

4. Wegwerfbarer Stecher nach Anspruch 2, **dadurch gekennzeichnet, daß** die Anschläge (11) als Widerhaken ausgebildet sind, die nach innen und nach vorne zeigen.

5. Wegwerfbarer Stecher nach einem der Ansprüche 1 bis 4, **dadurch gekennzeichnet, daß** der oder jeder Finger (9) von dem Lanzettenkörper nach außen geneigt ist und sich nach hinten erstreckt.

6. Wegwerfbarer Stecher nach einem der Ansprüche 1 bis 4, **dadurch gekennzeichnet, daß** jeder Finger (9) im allgemeinen parallel zu dem Körper der Lanzette und mit Abstand von diesem getrennt liegt, wobei jedoch seine Spitze (10) sich nach außen erweitert.

7. Wegwerfbarer Stecher nach einem der Ansprüche 1 bis 4, **dadurch gekennzeichnet, daß** die Nadelspitze (3) anfänglich durch eine längere Kappe (4) geschützt ist, durch die die Lanzette (2) aus einer anfänglichen Vorabschußposition, in der sich die oder jede Fingerspitze unmittelbar hinter dem zugehörigen Anschlag befindet, in die vollständig zurückgezogene Position gedrückt werden kann.

## Revendications

1. Dispositif jetable à piquer la peau comprenant un boîtier allongé (1) avec une lancette (2) mobile longitudinalement, chargée par ressort, renfermée dans celui-ci, la pointe de lancette (3) étant normalement à l'intérieur du boîtier (1), un mécanisme de gâchette (13) pour retenir la lancette dans une position complètement rétractée dans laquelle elle fournit de l'énergie au ressort (5), le mécanisme de gâchette étant actionnable pour déclencher la lancette pour amener la pointe à prendre une position momentanée faisant saillie à partir de l'extrémité avant du boîtier, et des moyens pour empêcher une utilisation répétée comprenant un doigt élastique (9) s'étendant vers l'arrière à partir de la lancette, le long mais espacé du corps de celle-ci, et une butée (11) sur l'intérieur du boîtier, derrière laquelle la pointe (10) du doigt (9) s'encliquette lorsque la lancette est déclenchée, **caractérisé par le fait qu'**une ouverture (14) est disposée dans la paroi latérale du boîtier (1), ce par quoi toute tentative pour repousser la lancette avec une force supérieure à une force prédéterminée après le déclenchement amène la butée (11) à dévier la pointe de doigt (10) vers à l'extérieur à travers ladite ouverture (14).

2. Dispositif à piquer jetable selon la revendication 1, dans lequel la lancette (2) est symétrique, avec deux doigts (9) sur des côtés opposés de celle-ci.

3. Dispositif à piquer jetable selon la revendication 2, dans lequel le boîtier comprend deux ouvertures opposées (14) et deux butées (11).

4. Dispositif à piquer jetable selon la revendication 2, dans lequel les butées (11) se présentent sous la forme d'ardillons dirigés vers l'intérieur et vers l'avant.

5. Dispositif à piquer jetable selon l'une quelconque des revendications 1 à 4, dans lequel le ou chaque doigt (9) s'incline vers l'extérieur à partir du corps de lancette tout en s'étendant vers l'arrière.

6. Dispositif à piquer jetable selon l'une quelconque des revendications 1 à 4, dans lequel le ou chaque doigt (9) est généralement parallèle à et espacée de la partie adjacente du corps de la lancette mais avec sa pointe (10) s'évasant vers l'extérieur.

7. Dispositif à piquer jetable selon l'une quelconque des revendication 1 à 4, dans lequel la pointe d'aiguille (3) est protégée initialement par un capuchon allongé (4) par lequel la lancette (2) peut être repoussée jusqu'à la position complètement rétractée depuis une position pré-déclenchée initiale dans laquelle la ou chaque pointe de doigt (10) est immédiatement à l'arrière de la butée associée.
